(19) Europäisches Patentamt
European Patent Office
Office européen des brevets

(11) EP 3 875 025 B1

(12) EUROPEAN PATENT SPECIFICATION

(45) Date of publication and mention of the grant of the patent:
**06.12.2023 Bulletin 2023/49**

(21) Application number: **19880328.0**

(22) Date of filing: **24.10.2019**

(51) International Patent Classification (IPC):
**A61B 5/352** $^{(2021.01)}$  **A61B 5/0245** $^{(2006.01)}$

(52) Cooperative Patent Classification (CPC):
**A61B 5/7207; A61B 5/0245; A61B 5/352**

(86) International application number:
**PCT/JP2019/041644**

(87) International publication number:
**WO 2020/090606 (07.05.2020 Gazette 2020/19)**

(54) **HEART RATE DETECTION METHOD, HEART RATE DETECTION DEVICE AND PROGRAM**

VERFAHREN ZUR ERFASSUNG DER HERZFRQUENZ, VORRICHTUNG ZUR ERFASSUNG DER HERZFRQUENZ UND PROGRAMM

PROCÉDÉ DE DÉTECTION DE FRÉQUENCE CARDIAQUE, DISPOSITIF DE DÉTECTION DE FRÉQUENCE CARDIAQUE ET PROGRAMME

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.10.2018 JP 2018203544**

(43) Date of publication of application:
**08.09.2021 Bulletin 2021/36**

(73) Proprietor: **NIPPON TELEGRAPH AND TELEPHONE CORPORATION**
**Chiyoda-ku, Tokyo 100-8116, (JP)**

(72) Inventors:
• **HASHIMOTO, Yuki**
**Musashino-shi, Tokyo 180-8585 (JP)**
• **KUWABARA, Kei**
**Musashino-shi, Tokyo 180-8585 (JP)**
• **MATSUURA, Nobuaki**
**Musashino-shi, Tokyo 180-8585 (JP)**

(74) Representative: **Samson & Partner Patentanwälte mbB**
**Widenmayerstraße 6**
**80538 München (DE)**

(56) References cited:
| | |
|---|---|
| CN-A- 105 411 579 | JP-A- 2008 104 641 |
| JP-U- S 505 491 | US-A1- 2007 255 150 |
| US-A1- 2009 082 682 | US-A1- 2009 171 228 |
| US-A1- 2016 074 666 | |

**Description**

Technical Field

The present invention relates to a heartbeat detection method, heartbeat detection device, and program for detecting a heartbeat (R wave) from an electrocardiographic waveform.

Background Art

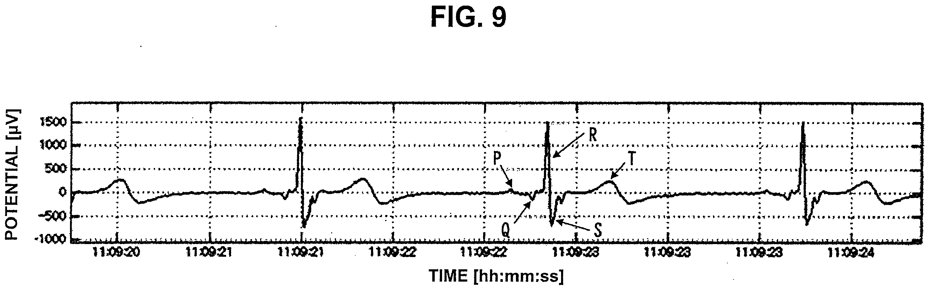

An ECG (Electrocardiogram) waveform is obtained by recording continuous electrical activities of a heart. A general ECG waveform is mainly formed from components called P, Q, R, S, and T waves representing the electrical activity statuses of right and left atriums and ventricles, as shown in Fig. 9. Biological information such as a heart rate obtained from an ECG waveform is used as a representative index value indicating exercise intensity in a sport or the activity status of the autonomic function in daily life.

As a method of detecting a heartbeat from an ECG waveform, a method of detecting the peak of an R wave having a relatively large amplitude from time-series data of the ECG waveform is easy. That is, with respect to the time-series data of the ECG waveform, a threshold is set in accordance with the amplitude of an R wave, an R wave is detected when a data value exceeds this threshold, and then an instantaneous heart rate is calculated from the period (patent literature 1).

To reduce undulation in a baseline of an ECG waveform caused by a body movement or the like, there are proposed a method (patent literature 2) that uses the time difference of the waveform as an index value instead of using the time-series data of the waveform, and a method (patent literature 3) that uses, as a new index, a value considering clearances before and after the peak from the time difference by paying attention to small individual differences of the peak widths between the Q, R, and S waves, thereby making it possible to detect an R wave more accurately.

In measurement of an ECG waveform by a wearable device that is attracting attention in monitoring of biological information at normal time or during exercise, electrical noise caused by floating of an electrode, a body movement, or myoelectricity may be added to an ECG waveform.

To cope with this, in a heartbeat detection method disclosed in patent literature 4, an abrupt variation in threshold is suppressed by setting, if large noise is superimposed on an ECG waveform, an upper limit for the above-described index value for R-wave detection, and not updating the threshold when the index value exceeds the upper limit, thereby detecting an R wave appropriately.

However, in the method disclosed in patent literature 4, if noise having an equal amplitude occurs between R waves, this noise is erroneously detected as an R wave, and a heart rate is thus erroneously detected as a value higher than an actual value.

Related Art Literature

Patent Literature

    Patent Literature 1: Japanese Patent Laid-Open No. 2015-156936
    Patent Literature 2: Japanese Patent Laid-Open No. 2017-29628
    Patent Literature 3: Japanese Patent Laid-Open No. 2017-42388
    Patent Literature 4: Japanese Patent Laid-Open No. 2017-150156

US 2009/0082682 A1 relates to methods and apparatus for detecting cardiac events based on heart rate sensitive parameters and discloses a system for the detection of cardiac events occurring in a human patient.

US 2009/0171228 A1 relates to baseline processing for the detection of cardiac events and also discloses a system for the detection of cardiac events occurring in a human patient.

Disclosure of Invention

Problem to be Solved by the Invention

The present invention has been made in consideration of the above problems, and has as its object to provide a heartbeat detection method, a heartbeat detection device, and a program which can accurately detect a heartbeat from an ECG waveform in which biological information other than a heartbeat represented by a body movement or myoelectricity is often mixed.

Means of Solution to the Problem

According to the present invention, there is provided a heartbeat detection method as defined in appended independent claims 1-5, said method comprising a first step of calculating a heartbeat time from a sampling data string of an electrocardiographic waveform of a living body, a second step of calculating, for each heartbeat time, a heart rate from the heartbeat time calculated in the first step, and a third step of calculating, based on the heart rate calculated in the second step, a length of a skip period every time the heartbeat time is calculated, wherein the first step includes a step in which if a time difference between a latest time calculated from the sampling data string and an immediately preceding heartbeat time is not longer than the length of the skip period calculated from the immediately preceding heartbeat time, the latest time calculated from the sampling data string is not adopted as a heartbeat time.
According to the present invention, there is also provided a heartbeat detection device as defined in appended independent claims 6-10, said device comprising a heartbeat time calculation unit configured to calculate a heartbeat time from a sampling data string of an electrocardiographic waveform of a living body, a heart rate calculation unit configured to calculate, for each heartbeat time, a heart rate from the heartbeat time calculated by the heartbeat time calculation unit, and a skip period calculation unit configured to calculate, based on the heart rate calculated by the heart rate calculation unit, a length of a skip period every time the heartbeat time is calculated, wherein if a time difference between a latest time calculated from the sampling data string and an immediately preceding heartbeat time is not longer than the length of the skip period calculated from the immediately preceding heartbeat time, the heartbeat time calculation unit does not adopt, as a heartbeat time, the latest time calculated from the sampling data string.
According to the present invention, there is also provided a heartbeat detection program causing a computer to execute the heartbeat detection method of the present invention.

Effect of the Invention

According to the present invention, a heart rate is calculated, for each heartbeat time, from the calculated heartbeat time, and the length of a skip period is calculated based on the heart rate. If a time difference between the latest time calculated from a sampling data string and an immediately preceding heartbeat time is equal to or shorter than the length of the skip period calculated from the immediately preceding heartbeat time, the latest time calculated from the sampling data string is not adopted as a heartbeat time, thereby making it possible to accurately detect a heartbeat even from an electrocardiographic waveform in which information other than the heartbeat is often mixed.

Brief Description of Drawings

Fig. 1 is a block diagram showing the arrangement of a heartbeat detection device according to an embodiment of the present invention;
Fig. 2 is a flowchart for explaining a heartbeat detection method according to the embodiment of the present invention;
Fig. 3 is a block diagram showing an example of the arrangement of a heartbeat time calculation unit of the heartbeat detection device according to the embodiment of the present invention;
Fig. 4 is a block diagram showing another example of the arrangement of the heartbeat time calculation unit of the heartbeat detection device according to the embodiment of the present invention;
Fig. 5 is a block diagram showing still another example of the arrangement of the heartbeat time calculation unit of the heartbeat detection device according to the embodiment of the present invention;
Fig. 6 is a timing chart showing time-series data of index values calculated by a conventional heartbeat detection method;
Fig. 7 is a timing chart showing instantaneous heart rates calculated by the conventional heartbeat detection method and the heartbeat detection method according to the embodiment of the present invention;
Fig. 8 is a block diagram showing an example of the arrangement of a computer for implementing the heartbeat detection device according to the embodiment of the present invention; and
Fig. 9 is a timing chart showing a representative electrocardiographic waveform.

Best Mode for Carrying Out the Invention

[Principles of Invention]

A heartbeat detection method according to the present invention calculates a heartbeat time from time-series data of an ECG waveform of a living body. However, by providing a period (skip period) during which the latest time calculated from the time-series data is not adopted as a heartbeat time based on a heart rate (R-R interval) detected from the time-series data of the ECG waveform, it is possible to prevent erroneous detection of noise during the period.

More specifically, while the conventional method detects no R wave during a period corresponding to an R-R interval corresponding to the upper limit of a detected heart rate after detection of a heartbeat, the present invention variably sets a length $t_{skip}$ of a skip period in accordance with an instantaneous heart rate X [bpm] obtained from the latest R-R interval, as given by equation (1) below.

$$t_{skip} = \frac{60}{Y(X)} \, [sec] \qquad \dots (1)$$

where Y(X) [bpm] represents a variable corresponding to the value of the instantaneous heart rate X. In consideration of prevention of detection of an abnormal value exceeding the upper limit value of the heart rate, the variable Y(X) takes a value equal to or smaller than the upper limit (the R-R interval as the time interval between an R wave and an immediately preceding R wave) of the instantaneous heart rate X. In consideration of missing an R wave (n-1) times (n is a natural number), the variable Y(X) is calculated by:

$$Y(X) = nX \qquad \dots (2)$$

In consideration of a variation component $\Delta X$ of the instantaneous heart rate X caused by a variation in appearance interval of an R wave, the variable Y(X) may be calculated by:

$$Y(X) = n(X + \Delta X) \qquad \dots (3)$$

In equation (3), n represents a natural number and $\Delta X$ is a constant. For example, $\Delta X$ can experimentally be obtained in advance from the time-series data of the instantaneous heart rates X.

In consideration of a possible upper limit value $X_{max}$ of the heart rate X, the variable Y(X) may be calculated by equation (4) below.

$$Y(X) = \begin{cases} 2X \ (X \le \frac{X_{max}}{2}) \\ (1+r)X \ (\frac{X_{max}}{2} < X \le \frac{X_{max}}{1+r}) \\ X_{max} \ (\frac{X_{max}}{1+r} < X) \end{cases} \qquad \dots (4)$$

where r represents a constant satisfying $0 \le r < 1$. Equation (4) means that Y (X) = 2X for $X \le X_{max}/2$, Y (X) = (1 + r) X for $X_{max}/2 < X \le X_{max}/ (1 + r)$, and Y(X) = $X_{max}$ for X > $X_{max}/ (1 + r)$. Equation (5) below may be used instead of equation (4).

$$Y(X) = \begin{cases} (n+1)X \ (X \le \frac{X_{max}}{n+1}) \\ (n+r)X \ (\frac{X_{max}}{n+1} < X \le \frac{X_{max}}{n+r}) \\ (m+r)X \ (\frac{X_{max}}{m+1+r} < X \le \frac{X_{max}}{m+r}) \\ X_{max} \ (\frac{X_{max}}{1+r} < X) \end{cases} \qquad \dots (5)$$

In equation (5), n represents an integer of 2 or more, and m takes each integer from (n-1) to 1. For example, m = 3, 2, 1 is obtained for n = 4. Equation (5) means that Y(X) = (n + 1)X for $X \le X_{max}/(n + 1)$, Y(X) = (n + r) X for $X_{max}/(n + 1) < X \le X_{max}/ (n + r)$, Y (X) = (m + r) X for $X_{max}/(m + 1 + r) < X \le X_{max}/(m + r)$, and Y (X) = $X_{max}$ for X > $X_{max}/(1 + r)$. Equation (5) is an equation obtained when considering missing of n R waves, and Equation (4) corresponds to a case in which n = 1 set in equation (5). In consideration of missing of R waves, the variation component $\Delta X$ of the heart rate X, and the upper limit value $X_{max}$ of the heart rate X, the variable Y(X) may be calculated by equation (6) below.

$$Y(X) = \begin{cases} 2(X + \Delta X)(X + \Delta X \leq \frac{X_{max}}{2}) \\ (1 + r)(X + \Delta X)(\frac{X_{max}}{2} < X + \Delta X \leq \frac{X_{max}}{1+r}) \\ X_{max} (\frac{X_{max}}{1+r} < X + \Delta X) \end{cases} \quad \ldots (6)$$

Equation (6) means that Y(X) = 2(X + $\Delta$X) for X + $\Delta$X $\leq$ $X_{max}$/2, Y (X) = (1 + r) (X + $\Delta$X) for $X_{max}$/2 < X + $\Delta$X $\leq$ $X_{max}$/ (1 + r), and Y (X) = $X_{max}$ for X + $\Delta$X > $X_{max}$/(1 + r) . Note that equation (7) below may be used instead of equation (6).

$$Y(X) = \begin{cases} (n+1)(X+\Delta X) \ (X+\Delta X \leq \frac{X_{max}}{n+1}) \\ (n+r)(X+\Delta X) \ (\frac{X_{max}}{n+1} < X+\Delta X \leq \frac{X_{max}}{n+r}) \\ (n+r)(X+\Delta X) \ (\frac{X_{max}}{n+1} < X+\Delta X \leq \frac{X_{max}}{n+r}) \\ X_{max} \ (\frac{X_{max}}{1+r} < X+\Delta X) \end{cases} \quad \ldots (7)$$

In equation (7), n represents an integer of 2 or more. Equation (7) means that Y(X) = (n + 1)(X + $\Delta$X) for X + $\Delta$X $\leq$ $X_{max}$/(n + 1), Y(X) = (n + r) (X + $\Delta$X) for $X_{max}$/(n + 1) < X + $\Delta$X $\leq$ $X_{max}$/(n + r), Y (X) = (m + r) (X + $\Delta$X) for $X_{max}$/(m + 1 + r) < X + $\Delta$X $\leq$ $X_{max}$/(m + r), and Y (X) = $X_{max}$ for X + $\Delta$X > $X_{max}$/(1 + r). Equation (7) is an equation obtained when considering missing of n R waves, and equation (6) corresponds to a case in which n = 1 is set in equation (7).

Note that if the variation component $\Delta$X of the heart rate X and the upper limit value $X_{max}$ of the heart rate X are considered without considering missing of R waves, the following equation is obtained.

$$Y(X) = \begin{cases} X + \Delta X \ (X + \Delta X \leq X_{max}) \\ X_{max} \ (X_{max} < X + \Delta X) \end{cases} \quad \ldots (8)$$

Equation (8) means that Y(X) = X + $\Delta$X for X + $\Delta$X $\leq$ $X_{max}$, and Y (X) = $X_{max}$ for X + $\Delta$X > $X_{max}$.

X in equations (2) to (8) need not always represent the instantaneous heart rate. As disclosed in Japanese Patent Laid-Open No. 2018-011819, for example, X may represent an average heart rate calculated based on time-series data of the instantaneous heart rates.

According to Japanese Patent Laid-Open No. 2018-011819, when HR(i) represents the ith instantaneous heart rate before averaging processing, X(i-1) represents a value obtained by averaging instantaneous heart rates up to the (i-1)th instantaneous heart rate, and q represents a predetermined averaging factor, the average heart rate X(i) obtained by averaging the instantaneous heart rates up to the ith instantaneous heart rate can be obtained by:

$$X(i) = q \times HR(i) + (1 - q) \times X(i-1) \quad \ldots (9)$$

As described above, according to the present invention, by variably setting, for each heartbeat detection operation, the skip period $t_{skip}$ during which no detection is performed (not adopted as a heartbeat time) when detecting a heartbeat, it is possible to prevent abnormal noise generated during the skip period $t_{skip}$ from being erroneously detected as a heartbeat.

Note that the heartbeat time in the present invention indicates a time at which a heartbeat of the heart of a living body is considered to occur.

[Embodiment]

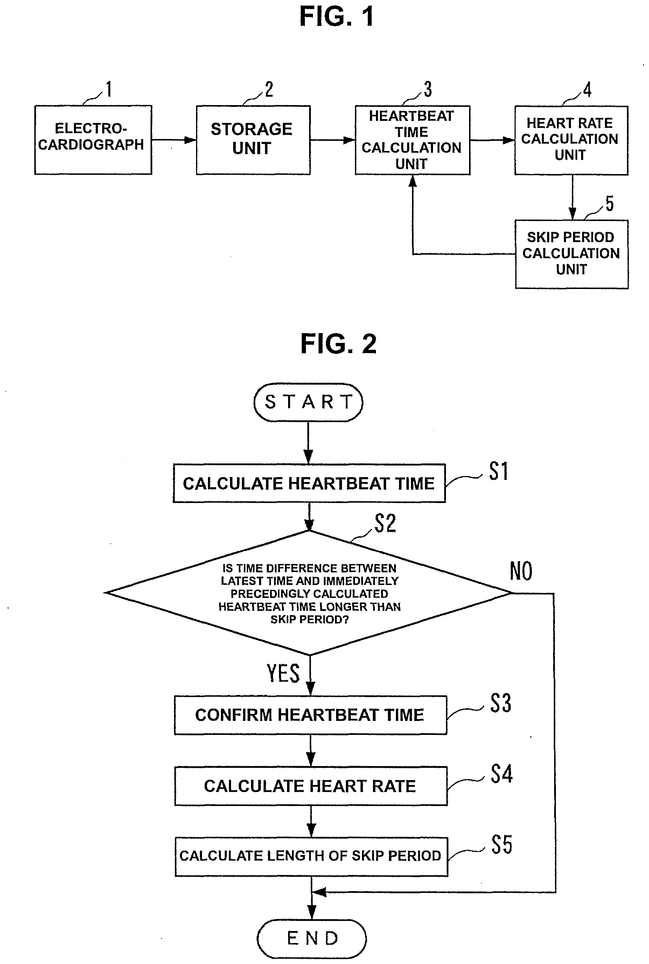

An embodiment of the present invention will be described below with reference to the accompanying drawings. Fig. 1 is a block diagram showing the arrangement of a heartbeat detection device according to the embodiment of the present invention. Fig. 2 is a flowchart for explaining a heartbeat detection method according to the embodiment. The heartbeat detection device includes an electrocardiograph 1 that outputs a sampling data string of an ECG waveform, a storage unit 2 that stores the sampling data string of the ECG waveform and sampling time information, a heartbeat time calculation unit 3 that calculates a heartbeat time from the sampling data string of the ECG waveform, a heart rate calculation unit 4 that calculates, for each heartbeat time, a heart rate X from the heartbeat time calculated by the heartbeat time calculation unit 3, and a skip period calculation unit 5 that calculates, every time the heartbeat time is calculated, the length of a skip period $t_{skip}$ based on the heart rate X calculated by the heart rate calculation unit 4.

The heartbeat detection method according to this embodiment will be described below. A procedure of detecting one heartbeat and obtaining the heartbeat time thereof will be explained. This heartbeat time calculation processing is repeated for the period of ECG waveform data, thereby obtaining the time-series data of the heartbeat times.

In this embodiment, a data string obtained by sampling an ECG waveform is represented by D(i) where i (i = 1, 2,...) is a number added to one sampling data. As the number i is larger, the sampling time is later, as a matter of course.

The electrocardiograph 1 measures the ECG waveform of a living body (human body) (not shown), and outputs the sampling data string D(i) of the ECG waveform. At this time, the electrocardiograph 1 adds sampling time information to each sampling data, and then outputs the sampling data string. Note that a practical measurement method of the ECG waveform is a well-known technique and a detailed description thereof will be omitted.

The storage unit 2 stores the sampling data string D(i) of the ECG waveform and the sampling time information, which have been output from the electrocardiograph 1.

Next, the heartbeat time calculation unit 3 calculates the heartbeat time from the sampling data string D(i) of the ECG waveform stored in the storage unit 2 (step S1 of Fig. 2).

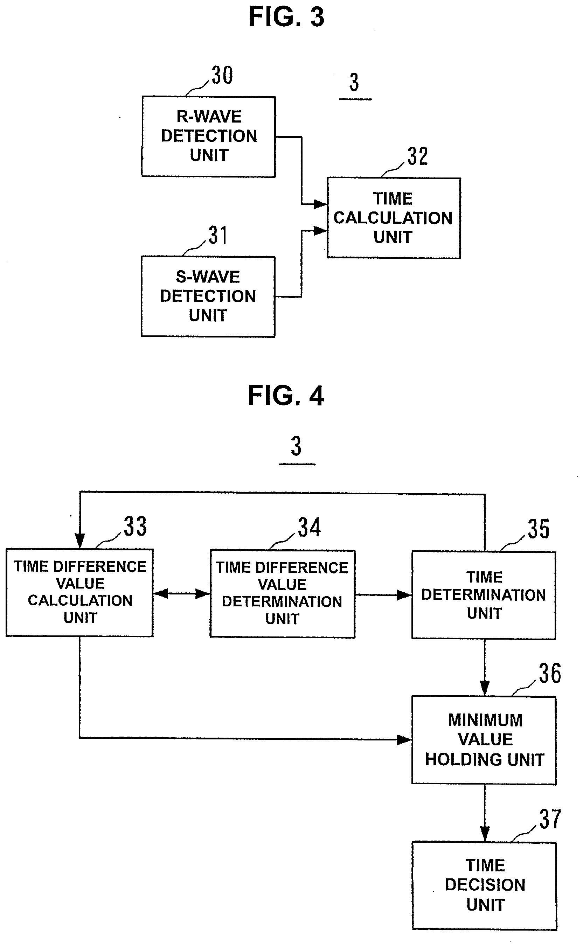

Fig. 3 is a block diagram showing an example of the arrangement of the heartbeat time calculation unit 3. The heartbeat time calculation unit 3 is formed from an R-wave detection unit 30 that detects sampling data as a representative point of an R wave by comparing the sampling data D(i) of the ECG waveform with a first threshold TH for identifying an R wave, an S-wave detection unit 31 that detects sampling data as a representative point of an S wave by comparing the sampling data D(i) of the ECG waveform with a second threshold TL (TH > TL) for identifying an S wave, and a time calculation unit 32 that detects sampling data of two points sandwiching a third threshold TM (TH > TM > TL) between the representative point of the R wave and that of the S wave existing immediately after the point and calculates, as a heartbeat time, a time at which a straight line connecting the sampling data of the two points intersects the third threshold TM.

Since the potential of the S or R wave changes depending on an ECG lead method, the threshold TL for identifying the S wave is appropriately set to a value of about 60% to 70% of the potential of the typical S wave of the lead method adopted by the electrocardiograph 1, and the threshold TH for identifying the R wave is appropriately set to a value of about 60% to 70% of the potential of the typical R wave of the lead method adopted by the electrocardiograph 1. The threshold TM is preferentially set to a value near the intermediate value between the thresholds TL and TH.

The arrangement of the heartbeat time calculation unit 3 shown in Fig. 3 is disclosed in patent literature 1 and a detailed description thereof will be omitted.

Fig. 4 is a block diagram showing another example of the arrangement of the heartbeat time calculation unit 3. The heartbeat time calculation unit 3 shown in Fig. 4 is formed from a time difference value calculation unit 33 that calculates, for each sampling time, the time difference value of the sampling data D(i) of the ECG waveform, a time difference value determination unit 34 that determines whether the time difference value is smaller than the threshold TH2, a time determination unit 35 that determines whether the first elapsed time from the immediately preceding heartbeat time to the latest sampling time at which the time difference value is obtained falls within the range of the first time interval, whether the second elapsed time from a time at which the time difference value becomes smaller than the threshold TH2 to the latest sampling time at which the time difference value is obtained falls within the range of the second time interval, and whether the third elapsed time from a time at which it is determined that the second elapsed time exceeds the range of the second time interval to the latest sampling time at which the time difference value is obtained falls within the range of the third time interval, a minimum value holding unit 36 that holds a minimum value Mini of the time difference values when the first elapsed time falls within the range of the first time interval, a minimum value Min2 of the time difference values when the second elapsed time falls within the range of the second time interval, and a minimum value Min3 of the time difference values when the third elapsed time falls within the range of the third time interval, and a time decision unit 37 that sets, if the relationship among the minimum values Mini, Min2, and Min3 satisfies a predetermined heartbeat time confirmation condition, as a heartbeat time, a time at which the time difference value becomes smaller than the threshold TH2 or the minimum value Min2 is obtained.

The time difference value calculation unit 33 acquires, from the storage unit 2, data D(i+1) one sampling operation after the sampling data D(i) and data D(i-1) one sampling operation before the sampling data D(i), and calculates a time difference value DY(i) of the sampling data D(i), as given by:

$$\text{DY(i)} = \text{D(i+1)} - \text{D(i-1)} \qquad\qquad ...(10)$$

The time difference value determination unit 34 determines whether the time difference value DY(i) is smaller than the threshold TH2. Since the peak of the time difference value DY(i) by an abrupt change from an R wave to an S wave is to be detected, this peak appears as a negative value. Therefore, the threshold TH2 is a negative value. The first time interval defines a time domain before the assumed next heartbeat time, the second time interval defines a time domain assumed to include the peak of the time difference value DY(i), and the third time interval defines a predetermined time domain after the time domain assumed to include the peak of the time difference value DY(i).

The time determination unit 35 sets, as the range of the first time interval, an interval from a time 150 ms shorter than an R-R interval obtained from the immediately preceding heartbeat time to a time obtained by adding 100 ms to the time. The R-R interval indicates a time obtained by subtracting the second preceding heartbeat time from the immediately preceding heartbeat time. Alternatively, the time determination unit 35 sets, as the range of the first time interval, the time domain from the immediately preceding heartbeat time to a time immediately before the time difference value DY(i) exceeds the threshold TH2 next. The second time interval preferably has a time width enough to cover the peak of the time difference value, and is preset to, for example, 50 ms. The third time interval is preset to, for example, 100 ms.

Furthermore, a condition that a ratio Min2/Min1 of the minimum value Min2 to the minimum value Mini and a ratio Min2/Min3 of the minimum value Min2 to the minimum value Min3 exceed a predetermined value is set as the heartbeat time confirmation condition.

The arrangement of the heartbeat time calculation unit 3 shown in Fig. 4 is disclosed in patent literature 2 and a detailed description thereof will be omitted.

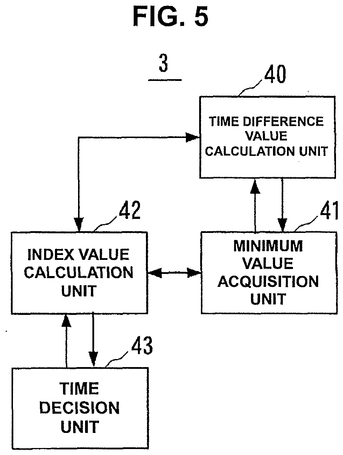

Fig. 5 is a block diagram showing still another example of the arrangement of the heartbeat time calculation unit 3. The heartbeat time calculation unit 3 shown in Fig. 5 is formed from a time difference value calculation unit 40 that calculates, for each sampling time, the time difference value DY(i) of the sampling data D(i) of the ECG waveform, a minimum value acquisition unit 41 that acquires, for each sampling point i, the minimum value of the time difference values in the predetermined time domains before and after the sampling point, an index value calculation unit 42 that obtains, for each sampling point i, as an index value, a value by subtracting the minimum value of the time difference values in the predetermined time domains before and after the sampling point i from the time difference value DY(i) of the sampling point i, and a time decision unit 43 that specifies, as a downward peak, from index values for the sampling points i, the index value of a point at which the index value becomes smaller than the predetermined threshold and the tendency of a change in index value changes from decrease to increase, and sets the time of the specified downward peak as a heartbeat time.

The predetermined time domains before and after the sampling point i are, for example, a domain of -112.5 ms to -12.5 ms and a domain of +12.5 ms to +112.5 ms with respect to the time of the sampling point i.

The arrangement of the heartbeat time calculation unit 3 shown in Fig. 5 is disclosed in patent literatures 3 and 4, and a detailed description thereof will be omitted.

Subsequently, the heartbeat time calculation unit 3 determines whether the time calculated in step S1 is appropriate, and confirms a heartbeat time. More specifically, the heartbeat time calculation unit 3 determines whether a time difference $\Delta T$ between the latest time calculated in step S1 and an immediately precedingly calculated/confirmed heartbeat time is longer than the length $t_{skip}$ of the skip period immediately precedingly calculated by the skip period calculation unit 5 (step S2 of Fig. 2). If the time difference $\Delta T$ is equal to or shorter than the length $t_{skip}$ of the skip period (NO in step S2), the time calculated in step S1 is discarded without being adopted as a heartbeat time. In this case, the processing target is advanced to the sampling data D(i) of the next sampling time, and the processes in step S1 and the subsequent steps are performed again.

Furthermore, if the time difference $\Delta T$ is longer than the length $t_{skip}$ of the skip period (YES in step S2), the heartbeat time calculation unit 3 determines the time calculated in step S1 as a heartbeat time (step S3 of Fig. 2).

Next, the heart rate calculation unit 4 calculates the heart rate X [bpm] from the latest heartbeat time calculated/confirmed by the heartbeat time calculation unit 3 (step S4 of Fig. 2). When the R-R interval as the time obtained by subtracting the immediately preceding heartbeat time from the latest heartbeat time calculated/confirmed by the heartbeat time calculation unit 3 is represented by RRI [ms], the heart rate calculation unit 4 calculates the instantaneous heart rate X by:

$$X = 60000/RRI \qquad \qquad ...(11)$$

The heart rate calculation unit 4 may calculate the average heart rate X by equation (9), instead of the instantaneous heart rate.

Subsequently, the skip period calculation unit 5 calculates the length $t_{skip}$ of the skip period by equation (1) and one of equations (2) to (8) based on the heart rate X (instantaneous heart rate or average heart rate) calculated by the heart rate calculation unit 4 (step S5 of Fig. 2). Note that the calculated length $t_{skip}$ of the skip period is used when the processing in step S2 is performed next.

The time-series data of the heartbeat times are obtained by repeating the processes in step S1 to S5.

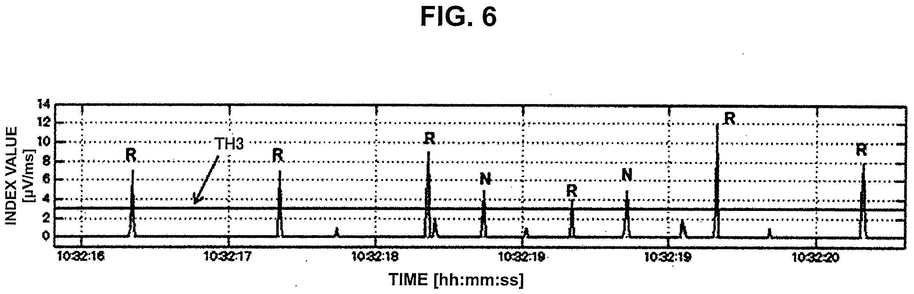

Fig. 6 shows the time-series data of index values (index values calculated by the index value calculation unit 42 of Fig. 5) calculated by the method disclosed in patent literature 3 from the time-series data of the ECG waveform. Note that a value obtained by subtracting the time difference value DY(i) of the sampling point i from the minimum value of the time difference values in the predetermined time domains before and after the sampling point i is set as an index value. In Fig. 6, R represents an R wave and N represents noise other than the R waves. In the example shown in Fig. 6, two large noise components (N) appear in the index values between the third and fifth R waves, and the two noise components exceed a threshold TH3. Thus, it is understood that the times of the two noise components are erroneously calculated as heartbeat times.

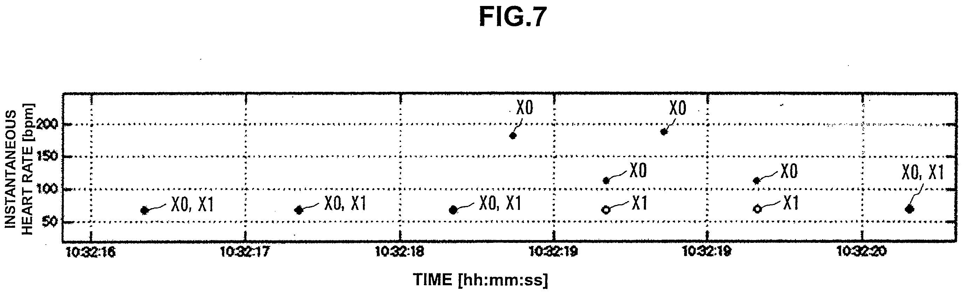

Fig. 7 shows the instantaneous heart rates calculated by the conventional heartbeat detection method disclosed in patent literature 4 and by this embodiment based on the time-series data of the same ECG waveform as in Fig. 6. In Fig. 7, X0 represents the instantaneous heart rate calculated by the conventional heartbeat detection method and X1 represents the instantaneous heart rate calculated by this embodiment. In this embodiment, n is set to 1 (equation (2)), $\Delta X$ is set to 15 bpm, r is set to 1/3, and $X_{max}$ is set to 250 bpm with reference to patent literature 3. In the conventional heartbeat detection method, since the time of noise is also detected as a heartbeat time, the instantaneous heart rate changes irregularly. On the other hand, in this embodiment, the instantaneous heart rate is stable, and it is thus understood that erroneous calculation of the instantaneous heart rate caused by noise can be prevented.

As described above, according to this embodiment, it is demonstrated that it is possible to accurately detect a heartbeat even from an ECG waveform in which information other than the heartbeat (R wave) is often mixed.

Note that in this embodiment, the methods disclosed in patent literature 1 to 4 are each used as the heartbeat time calculation method. The present invention, however, is applicable regardless of the heartbeat time calculation method.

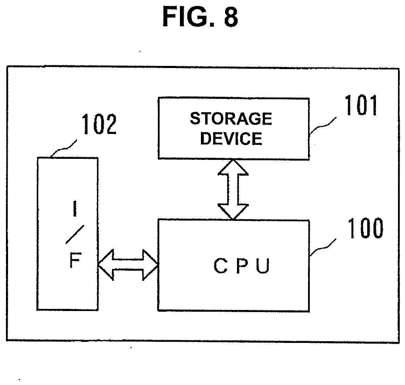

The storage unit 2, heartbeat time calculation unit 3, heart rate calculation unit 4, and skip period calculation unit 5 of the heartbeat detection device described in this embodiment can be implemented by a computer including a CPU (Central Processing Unit), a storage device, and an interface, and a program for controlling these hardware resources. Fig. 8 shows an example of the arrangement of this computer. The computer includes a CPU 100, a storage device 101, and an interface device (to be referred to as an I/F hereinafter) 102. The I/F 102 is connected to the electrocardiograph 1 and the like. In this computer, a heartbeat detection program for implementing the heartbeat detection method of the present invention is provided while being recorded on a recording medium such as a flexible disk, CD-ROM, DVD-ROM, or memory card, and stored in the storage device 101. The CPU 100 executes the processing described in this embodiment in accordance with the program stored in the storage device 101.

Industrial Applicability

The present invention is applicable to a technique of detecting a heartbeat of a living body. Explanation of the Reference Numerals and Signs

1... electrocardiograph, 2...storage unit, 3...heartbeat time calculation unit, 4...heart rate calculation unit, 5...skip period calculation unit, 30...R-wave detection unit, 31...S-wave detection unit, 32...time calculation unit, 33, 40...time difference value calculation unit, 34...time difference value determination unit, 35...time determination unit, 36...minimum value holding unit, 37, 43...time decision unit, 41...minimum value acquisition unit, 42...index value calculation unit

**Claims**

1. A computer-implemented heartbeat detection method comprising:

   a first step (S1) of calculating a heartbeat time from a sampling data string of an electrocardiographic waveform of a living body;
   a second step (S4) of calculating, for each heartbeat time, a heart rate from the heartbeat time calculated in the first step; and
   a third step (S5) of calculating, based on the heart rate calculated in the second step, a length of a skip period every time the heartbeat time is calculated,
   wherein the first step includes a step in which if a time difference between a latest time calculated from the sampling data string and an immediately preceding heartbeat time is not longer than the length of the skip period calculated from the immediately preceding heartbeat time, the latest time calculated from the sampling data string is not adopted as a heartbeat time, **characterized in that**
   the third step includes a step in which a variable Y(X) is calculated such that $Y(X) - 2(X + \Delta X)$ if $X + \Delta X \leq X_{max}/2$, $Y(X) = (1 + r)(X + \Delta X)$ if $X_{max}/2 < X + \Delta X \leq X_{max}/(1 + r)$, and $Y(X) = Y_{max}$ if $X + \Delta X > X_{max}/(1 + r)$, and the length of the skip period is calculated based on a reciprocal of the variable Y(X), wherein X represents the heart

rate, $X_{max}$ represents a predetermined possible upper limit value of the heart rate X, $\Delta X$ is a constant which represents a variation component of the heart rate X caused by a variation in appearance interval of an R wave, and r is a constant between not less than 0 and less than 1.

2. A computer-implemented heartbeat detection method comprising:

a first step (S1) of calculating a heartbeat time from a sampling data string of an electrocardiographic waveform of a living body;

a second step (S4) of calculating, for each heartbeat time, a heart rate from the heartbeat time calculated in the first step; and

a third step (S5) of calculating, based on the heart rate calculated in the second step, a length of a skip period every time the heartbeat time is calculated,

wherein the first step includes a step in which if a time difference between a latest time calculated from the sampling data string and an immediately preceding heartbeat time is not longer than the length of the skip period calculated from the immediately preceding heartbeat time, the latest time calculated from the sampling data string is not adopted as a heartbeat time, **characterized in that**

the third step includes a step in which the variable Y(X) is calculated such that $Y(X) = 2X$ if $X \le X_{max}/2$, $Y(X) = (1 + r)X$ if $X_{max}/2 < X \le X_{max}/(1 + r)$, and $Y(X) = X_{max}$ if $X > X_{max}/(1 + r)$, and the length of the skip period is calculated based on a reciprocal of the variable Y(X), wherein X represents the heart rate, $X_{max}$ represents a predetermined possible upper limit value of the heart rate X, and r is a constant between not less than 0 and less than 1.

3. A computer-implemented heartbeat detection method comprising:

a first step (S1) of calculating a heartbeat time from a sampling data string of an electrocardiographic waveform of a living body;

a second step (S4) of calculating, for each heartbeat time, a heart rate from the heartbeat time calculated in the first step; and

a third step (S5) of calculating, based on the heart rate calculated in the second step, a length of a skip period every time the heartbeat time is calculated,

wherein the first step includes a step in which if a time difference between a latest time calculated from the sampling data string and an immediately preceding heartbeat time is not longer than the length of the skip period calculated from the immediately preceding heartbeat time, the latest time calculated from the sampling data string is not adopted as a heartbeat time, **characterized in that**

the third step includes a step in which the variable Y(X) is calculated such that $Y(X) = (n + 1)X$ if $X \le X_{max}/(n + 1)$, $Y(X) = (n + r)X$ if $X_{max}/(n + 1) < X \le X_{max}/(n + r)$, $Y(X) = (m + r)X$ if $X_{max}/(m + 1 + r) < X \le X_{max}/(m + r)$, and $Y(X) = X_{max}$ if $> X_{max}/(1 + r)$, and the length of the skip period is calculated based on a reciprocal of the variable Y(X), wherein X represents the heart rate, $X_{max}$ represents a predetermined possible upper limit value of the heart rate X, n is an integer not less than 2, m represents each integer from 1 to n-1, and r is a constant between not less than 0 and less than 1.

4. A computer-implemented heartbeat detection method comprising:

a first step (S1) of calculating a heartbeat time from a sampling data string of an electrocardiographic waveform of a living body;

a second step (S4) of calculating, for each heartbeat time, a heart rate from the heartbeat time calculated in the first step; and

a third step (S5) of calculating, based on the heart rate calculated in the second step, a length of a skip period every time the heartbeat time is calculated,

wherein the first step includes a step in which if a time difference between a latest time calculated from the sampling data string and an immediately preceding heartbeat time is not longer than the length of the skip period calculated from the immediately preceding heartbeat time, the latest time calculated from the sampling data string is not adopted as a heartbeat time, **characterized in that**

the third step includes a step in which the variable Y(X) is calculated such that $Y(X) = (n + 1)(X + \Delta X)$ if $X + \Delta X \le X_{max}/(n + 1)$, $Y(X) = (n + r)(X + \Delta X)$ if $X_{max}/(n + 1) < X + \Delta X \le X_{max}/(n + r)$, $Y(X) = (m + r)(X + \Delta X)$ if $X_{max}/(m + 1 + r) < X + \Delta X \le X_{max}/(m + r)$, and $Y(X) = X_{max}$ if $X + \Delta X > X_{max}/(1 + r)$, and the length of the skip period is calculated based on a reciprocal of the variable Y(X), wherein X represents the heart rate, $X_{max}$ represents a predetermined possible upper limit value of the heart rate X, $\Delta X$ is a constant which represents a variation component of the heart rate X caused by a variation in appearance interval of an R wave, n is an integer not less than 2, m represents each integer from 1 to n-1, and r is a constant between not less than 0 and less than 1.

5. A computer-implemented heartbeat detection method comprising:

a first step (S1) of calculating a heartbeat time from a sampling data string of an electrocardiographic waveform of a living body;
a second step (S4) of calculating, for each heartbeat time, a heart rate from the heartbeat time calculated in the first step; and
a third step (S5) of calculating, based on the heart rate calculated in the second step, a length of a skip period every time the heartbeat time is calculated,
wherein the first step includes a step in which if a time difference between a latest time calculated from the sampling data string and an immediately preceding heartbeat time is not longer than the length of the skip period calculated from the immediately preceding heartbeat time, the latest time calculated from the sampling data string is not adopted as a heartbeat time, **characterized in that**
the third step includes a step in which the variable Y (X) is calculated such that $Y(X) = X + \Delta X$ if $X + \Delta X \leq X_{max}$, and $Y(X) = X_{max}$ if $X + \Delta X > X_{max}$, and the length of the skip period is calculated based on a reciprocal of the variable $Y(X)$, wherein X represents the heart rate, $X_{max}$ represents a predetermined possible upper limit value of the heart rate X, $\Delta X$ is a constant which represents a variation component of the heart rate X caused by a variation in appearance interval of an R wave.

6. A heartbeat detection device comprising:

a heartbeat time calculation unit (3) configured to calculate a heartbeat time from a sampling data string of an electrocardiographic waveform of a living body;
a heart rate calculation unit (4) configured to calculate, for each heartbeat time, a heart rate from the heartbeat time calculated by the heartbeat time calculation unit (3); and
a skip period calculation unit (5) configured to calculate, based on the heart rate calculated by the heart rate calculation unit (4), a length of a skip period every time the heartbeat time is calculated,
wherein if a time difference between a latest time calculated from the sampling data string and an immediately preceding heartbeat time is not longer than the length of the skip period calculated from the immediately preceding heartbeat time, the heartbeat time calculation unit (3) does not adopt, as a heartbeat time, the latest time calculated from the sampling data string, **characterized in that**
the skip period calculation unit (5) is configured to calculate a variable $Y(X)$ such that $Y(X) = 2(X + \Delta X)$ if $X + \Delta X \leq X_{max}/2$, $Y(X) = (1 + r)(X + \Delta X)$ if $X_{max}/2 < X + \Delta X \leq X_{max}/(1 + r)$, and $Y(X) = Y_{max}$ if $X + \Delta X > X_{max}/(1 + r)$, and to calculate the length of the skip period based on a reciprocal of the variable $Y(X)$, wherein X represents the heart rate, $X_{max}$ represents a predetermined possible upper limit value of the heart rate X, $\Delta X$ is a constant which represents a variation component of the heart rate X caused by a variation in appearance interval of an R wave, and r is a constant between not less than 0 and less than 1.

7. A heartbeat detection device comprising:

a heartbeat time calculation unit (3) configured to calculate a heartbeat time from a sampling data string of an electrocardiographic waveform of a living body;
a heart rate calculation unit (4) configured to calculate, for each heartbeat time, a heart rate from the heartbeat time calculated by the heartbeat time calculation unit (3); and
a skip period calculation unit (5) configured to calculate, based on the heart rate calculated by the heart rate calculation unit (4), a length of a skip period every time the heartbeat time is calculated,
wherein if a time difference between a latest time calculated from the sampling data string and an immediately preceding heartbeat time is not longer than the length of the skip period calculated from the immediately preceding heartbeat time, the heartbeat time calculation unit (3) does not adopt, as a heartbeat time, the latest time calculated from the sampling data string, **characterized in that**
the skip period calculation unit (5) is configured to calculate the variable $Y(X)$ such that $Y(X) = 2X$ if $X \leq X_{max}/2$, $Y(X) = (1 + r)X$ if $X_{max}/2 < X \leq X_{max}/(1 + r)$, and $Y(X) = X_{max}$ if $X > X_{max}/(1 + r)$, and to calculate the length of the skip period based on a reciprocal of the variable $Y(X)$, wherein X represents the heart rate, $X_{max}$ represents a predetermined possible upper limit value of the heart rate X, and r is a constant between not less than 0 and less than 1.

8. A heartbeat detection device comprising:

a heartbeat time calculation unit (3) configured to calculate a heartbeat time from a sampling data string of an electrocardiographic waveform of a living body;

a heart rate calculation unit (4) configured to calculate, for each heartbeat time, a heart rate from the heartbeat time calculated by the heartbeat time calculation unit (3); and

a skip period calculation unit (5) configured to calculate, based on the heart rate calculated by the heart rate calculation unit (4), a length of a skip period every time the heartbeat time is calculated,

wherein if a time difference between a latest time calculated from the sampling data string and an immediately preceding heartbeat time is not longer than the length of the skip period calculated from the immediately preceding heartbeat time, the heartbeat time calculation unit (3) does not adopt, as a heartbeat time, the latest time calculated from the sampling data string, **characterized in that**

the skip period calculation unit (5) is configured to calculate the variable $Y(X)$ such that $Y(X) = (n + 1)X$ if $X \leq X_{max}/(n + 1)$, $Y(X) = (n + r) X$ if $X_{max}/(n + 1) < X \leq X_{max}/(n + r)$, $Y (X) = (m + r)X$ if $X_{max}/(m + 1 + r) < X \leq X_{max}/(m + r)$, and $Y(X) - X_{max}$ if $X > X_{max}/(1 + r)$, and to calculate the length of the skip period based on a reciprocal of the variable $Y(X)$, wherein $X$ represents the heart rate, $X_{max}$ represents a predetermined possible upper limit value of the heart rate $X$, $n$ is an integer not less than 2, $m$ represents each integer from 1 to n-1, and $r$ is a constant between not less than 0 and less than 1.

9. A heartbeat detection device comprising:

a heartbeat time calculation unit (3) configured to calculate a heartbeat time from a sampling data string of an electrocardiographic waveform of a living body;

a heart rate calculation unit (4) configured to calculate, for each heartbeat time, a heart rate from the heartbeat time calculated by the heartbeat time calculation unit (3); and

a skip period calculation unit (5) configured to calculate, based on the heart rate calculated by the heart rate calculation unit (4), a length of a skip period every time the heartbeat time is calculated,

wherein if a time difference between a latest time calculated from the sampling data string and an immediately preceding heartbeat time is not longer than the length of the skip period calculated from the immediately preceding heartbeat time, the heartbeat time calculation unit (3) does not adopt, as a heartbeat time, the latest time calculated from the sampling data string, **characterized in that**

the skip period calculation unit (5) is configured to calculate the variable $Y(X)$ such that $Y(X) = (n + 1) (X + \Delta X)$ if $X + \Delta X \leq X_{max}/(n + 1)$, $Y (X) = (n + r) (X + \Delta X)$ if $X_{max}/(n + 1) < X + \Delta X \leq X_{max}/(n + r)$, $Y (X) = (m + r) (X + \Delta X)$ if $X_{max}/(m + 1 + r) < X + \Delta X \leq X_{max}/(m + r)$, and $Y(X) = X_{max}$ if $X + \Delta X > X_{max}/ (1 + r)$, and to calculate the length of the skip period based on a reciprocal of the variable $Y(X)$, wherein $X$ represents the heart rate, $X_{max}$ represents a predetermined possible upper limit value of the heart rate $X$, $\Delta X$ is a constant which represents a variation component of the heart rate $X$ caused by a variation in appearance interval of an R wave, $n$ is an integer not less than 2, $m$ represents each integer from 1 to n-1, and $r$ is a constant between not less than 0 and less than 1.

10. A heartbeat detection device comprising:

a heartbeat time calculation unit (3) configured to calculate a heartbeat time from a sampling data string of an electrocardiographic waveform of a living body;

a heart rate calculation unit (4) configured to calculate, for each heartbeat time, a heart rate from the heartbeat time calculated by the heartbeat time calculation unit (3); and

a skip period calculation unit (5) configured to calculate, based on the heart rate calculated by the heart rate calculation unit (4), a length of a skip period every time the heartbeat time is calculated,

wherein if a time difference between a latest time calculated from the sampling data string and an immediately preceding heartbeat time is not longer than the length of the skip period calculated from the immediately preceding heartbeat time, the heartbeat time calculation unit (3) does not adopt, as a heartbeat time, the latest time calculated from the sampling data string, **characterized in that**

the skip period calculation unit (5) is configured to calculate the variable $Y(X)$ such that $Y(X) = X + \Delta X$ if $X + \Delta X \leq X_{max}$, and $Y (X) = X_{max}$ if $X + \Delta X > X_{max}$, and to calculate the length of the skip period based on a reciprocal of the variable $Y(X)$, wherein $X$ represents the heart rate, $X_{max}$ represents a predetermined possible upper limit value of the heart rate $X$, $\Delta X$ is a constant which represents a variation component of the heart rate $X$ caused by a variation in appearance interval of an R wave.

11. A heartbeat detection program comprising instructions which, when executed on a computer, cause said computer to execute the method of anyone of claims 1-5.

**Patentansprüche**

1.  Computer-implementiertes Verfahren zur Detektion von Herzschlägen, umfassend:

    einen ersten Schritt (S1) des Berechnens eines Herzschlagzeitpunkts aus einer Abtastdatenreihe einer elektrokardiographischen Wellenform eines lebenden Körpers;
    einen zweiten Schritt (S4), in dem für jeden Herzschlagzeitpunkt eine Herzschlagrate aus dem in dem ersten Schritt berechneten Herzschlagzeitpunkt berechnet wird; und
    einen dritten Schritt (S5) des Berechnens, basierend auf der im zweiten Schritt berechneten Herzschlagrate, einer Länge einer Auslassungsperiode jedes Mal, wenn der Herzschlagzeitunkt berechnet wird,
    wobei der erste Schritt einen Schritt umfasst, bei dem, wenn eine Zeitdifferenz zwischen einem letzten aus der Abtastdatenreihe berechneten Zeitpunkt und einem unmittelbar vorhergehenden Herzschlagzeitpunkt nicht größer ist als die Länge der aus dem unmittelbar vorhergehenden Herzschlagzeitpunkt berechneten Auslassungsperiode, der letzte aus der Abtastdatenreihe berechnete Zeitpunkt nicht als Herzschlagzeitpunkt angenommen wird, **dadurch gekennzeichnet, dass**
    der dritte Schritt einen Schritt umfasst, in dem eine Variable Y(X) so berechnet wird, dass $Y(X) = 2(X + \Delta X)$, wenn $X + \Delta X \leq X_{max}/2$, $Y(X) - (1 + r)(X + \Delta X)$, wenn $X_{max}/2 < X + \Delta X \leq X_{max}/(1 + r)$, und $Y(X) = Y_{max}$, wenn $X + \Delta X > X_{max}/(1 + r)$, und die Länge der Auslassungsperiode wird auf der Grundlage eines Kehrwerts der Variablen Y(X) berechnet, wobei X die Herzschlagrate darstellt, $X_{max}$ einen vorbestimmten möglichen oberen Grenzwert der Herzschlagrate X darstellt, $\Delta X$ eine Konstante ist, die eine Variationskomponente der Herzschlagrate X darstellt, die durch eine Variation im Auftrittsintervall einer R-Welle verursacht wird, und r eine Konstante zwischen nicht weniger als 0 und weniger als 1 ist.

2.  Computerimplementiertes Verfahren zur Detektion von Herzschlägen, umfassend:

    einen ersten Schritt (S1) des Berechnens eines Herzschlagzeitpunkts aus einer Abtastdatenreihe einer elektrokardiographischen Wellenform eines lebenden Körpers;
    einen zweiten Schritt (S4) zum Berechnen einer Herzschlagrate für jeden Herzschlagzeitpunkt aus dem im ersten Schritt berechneten Herzschlagzeitpunkt; und
    einen dritten Schritt (S5) des Berechnens, basierend auf der im zweiten Schritt berechneten Herzschlagrate, einer Länge einer Auslassungsperiode jedes Mal, wenn der Herzschlagzeitpunkt berechnet wird,
    wobei der erste Schritt einen Schritt einschließt, in dem, wenn eine Zeitdifferenz zwischen einem letzten aus der Abtastdatenreihe berechneten Zeitpunkt und einem unmittelbar vorangehenden Herzschlagzeitpunkt nicht größer ist als die Länge der aus dem unmittelbar vorangehenden Herzschlagzeitpunkt berechneten Auslassungsperiode, der letzte aus der Abtastdatenreihe berechnete Zeitpunkt nicht als Herzschlagzeitpunkt angenommen wird, **dadurch gekennzeichnet, dass**
    der dritte Schritt einen Schritt umfasst, in dem die Variable Y(X) so berechnet wird, dass $Y(X) = 2X$, wenn $X \leq X_{max}/2$, $Y(X) - (1 + r)X$, wenn $X_{max}/2 < X \leq X_{max}/(1 + r)$, und $Y(X) = X_{max}$, wenn $X > X_{max}/(1 + r)$, und die Länge der Auslassungsperiode auf der Grundlage eines Kehrwerts der Variablen Y(X) berechnet wird, wobei X die Herzschlagrate darstellt, $X_{max}$ einen vorbestimmten möglichen oberen Grenzwert der Herzschlagrate X darstellt und r eine Konstante zwischen nicht weniger als 0 und weniger als 1 ist.

3.  Computer-implementiertes Verfahren zur Detektion von Herzschlägen, umfassend:

    einen ersten Schritt (S1) des Berechnens eines Herzschlagzeitpunkts aus einer Abtastdatenreihe einer elektrokardiographischen Wellenform eines lebenden Körpers;
    einen zweiten Schritt (S4) zum Berechnen einer Herzschlagrate für jeden Herzschlagzeitpunkt aus dem im ersten Schritt berechneten Herzschlagzeitpunkt; und
    einen dritten Schritt (S5) des Berechnens, basierend auf der im zweiten Schritt berechneten Herzschlagrate, einer Länge einer Auslassungsperiode jedes Mal, wenn der Herzschlagzeitpunkt berechnet wird,
    wobei der erste Schritt einen Schritt umfasst, in dem, wenn eine Zeitdifferenz zwischen einem letzten aus der Abtastdatenreihe berechneten Zeitpunkt und einem unmittelbar vorhergehenden Herzschlagzeitpunkt nicht größer ist als die Länge der aus dem unmittelbar vorhergehenden Herzschlagzeitpunkt berechneten Auslassungsperiode, der letzte aus der Abtastdatenreihe berechnete Zeitpunkt nicht als Herzschlagzeitpunkt angenommen wird, **dadurch gekennzeichnet, dass**

der dritte Schritt einen Schritt umfasst, in dem die Variable Y(X) so berechnet wird, dass $Y(X) = (n + 1)X$, wenn $X \leq X_{max}/(n + 1)$, $Y(X) = (n + r)X$, wenn $X_{max}/(n + 1) < X \leq X_{max}/(n + r)$, $Y(X) = (m + r)X$, wenn $X_{max}/(m + 1 + r) < X \leq X_{max}/(m + r)$, und $Y(X) = X_{max}$, wenn $X > X_{max}/(1 + r)$, und die Länge der Auslassungsperiode auf der Grundlage eines Kehrwerts der Variablen Y(X) berechnet wird, wobei X die Herzschlagrate darstellt, $X_{max}$ einen vorbestimmten möglichen oberen Grenzwert der Herzschlagrate X darstellt, n eine ganze Zahl nicht kleiner als 2 ist, m jede ganze Zahl von 1 bis n-1 darstellt und r eine Konstante zwischen nicht kleiner als 0 und kleiner als 1 ist.

4. Computer-implementiertes Verfahren zur Detektion von Herzschlägen, umfassend:

einen ersten Schritt (S1) des Berechnens eines Herzschlagzeitpunkts aus einer Abtastdatenreihe einer elektrokardiographischen Wellenform eines lebenden Körpers;
einen zweiten Schritt (S4), in dem für jeden Herzschlagzeitpunkt eine Herzfrequenz aus dem im ersten Schritt berechneten Herzschlagzeitpunkt berechnet wird; und
einen dritten Schritt (S5) des Berechnens, basierend auf der im zweiten Schritt berechneten Herzschlagrate, einer Länge einer Auslassungsperiode jedes Mal, wenn der Herzschlagzeitunkt berechnet wird,
wobei der erste Schritt einen Schritt umfasst, bei dem, wenn eine Zeitdifferenz zwischen einem letzten aus der Abtastdatenreihe berechneten Zeitpunkt und einem unmittelbar vorhergehenden Herzschlagzeitpunkt nicht länger ist als die Länge der aus dem unmittelbar vorhergehenden Herzschlagzeitpunkt berechneten Auslassungsperiode, der letzte aus der Abtastdatenreihe berechnete Zeitpunkt nicht als Herzschlagzeitpunkt angenommen wird, **dadurch gekennzeichnet, dass**
der dritte Schritt einen Schritt umfasst, in dem die Variable Y(X) so berechnet wird, dass $Y(X) = (n + 1)(X + \Delta X)$, wenn $X + \Delta X \leq X_{max}/(n + 1)$, $Y(X) = (n + r)(X + \Delta X)$, wenn $X_{max}/(n + 1) < X + \Delta X \leq X_{max}/(n + r)$, $Y(X) - (m + r)(X + \Delta X)$, wenn $X_{max}/(m + 1 + r) < X + \Delta X \leq X_{max}/(m + r)$, und $Y(X) = X_{max}$, wenn $X + \Delta X > X_{max}/(1 + r)$, und die Länge der Auslassungsperiode wird auf der Grundlage eines Kehrwertes der Variablen Y(X) berechnet, wobei X die Herzschlagrate darstellt, $X_{max}$ einen vorbestimmten möglichen oberen Grenzwert der Herzschlagrate X darstellt, $\Delta X$ eine Konstante ist, die eine Variationskomponente der Herzschlagrate X darstellt, die durch eine Variation im Auftrittsintervall einer R-Welle verursacht wird, n eine ganze Zahl nicht kleiner als 2 ist, m jede ganze Zahl von 1 bis n-1 darstellt, und r eine Konstante zwischen nicht kleiner als 0 und kleiner als 1 ist.

5. Computer-implementiertes Verfahren zur Detektion von Herzschlägen, umfassend:

einen ersten Schritt (S1) des Berechnens eines Herzschlagzeitpunkts aus einer Abtastdatenreihe einer elektrokardiographischen Wellenform eines lebenden Körpers; einen zweiten Schritt (S4) zum Berechnen einer Herzschlagrate für jeden Herzschlagzeitpunkt aus dem im ersten Schritt berechneten Herzschlagzeitpunkt; und
einen dritten Schritt (S5) des Berechnens, basierend auf der im zweiten Schritt berechneten Herzschlagrate, einer Länge einer Auslassungsperiode jedes Mal, wenn der Herzschlagzeitpunkt berechnet wird,
wobei der erste Schritt einen Schritt einschließt, in dem, wenn eine Zeitdifferenz zwischen einem letzten Zeitpunkt, der aus der Abtastdatenreihe berechnet wird, und einem unmittelbar vorhergehenden Herzschlagzeitpunkt nicht länger ist als die Länge der Auslassungsperiode, die aus dem unmittelbar vorhergehenden Herzschlagzeitpunkt berechnet wird, der letzte Zeitpunkt, der aus der Abtastdatenreihe berechnet wird, nicht als Herzschlagzeitpunkt angenommen wird, **dadurch gekennzeichnet, dass**
der dritte Schritt einen Schritt umfasst, in dem die Variable Y(X) so berechnet wird, dass $Y(X) - X + \Delta X$, wenn $X + \Delta X \leq X_{max}$, und $Y(X) = X_{max}$, wenn $X + \Delta X > X_{max}$, und die Länge der Auslassungsperiode auf der Grundlage eines Kehrwerts der Variablen Y(X) berechnet wird, wobei X die Herzschlagrate darstellt, $X_{max}$ einen vorbestimmten möglichen oberen Grenzwert der Herzschlagrate X darstellt, $\Delta X$ eine Konstante ist, die eine Variationskomponente der Herzschlagrate X darstellt, die durch eine Variation des Auftrittsintervalls einer R-Welle verursacht wird.

6. Herzschlag-Detektionsvorrichtung, umfassend:

eine Herzschlagzeitpunkt-Berechnungseinheit (3), die konfiguriert ist, um einen Herzschlagzeitpunkt aus einer Abtastdatenreihe einer elektrokardiographischen Wellenform eines lebenden Körpers zu berechnen;
eine Herzschlagraten-Berechnungseinheit (4), die konfiguriert ist, um für jeden Herzschlagzeitpunkt eine Herzschlagrate aus dem von der Herzschlagzeitpunkt-Berechnungseinheit (3) berechneten Herzschlagzeitpunkt zu berechnen; und
eine Auslassungsperioden-Berechnungseinheit (5), die konfiguriert ist, um auf der Grundlage der von der Herzschlagraten-Berechnungseinheit (4) berechneten Herzschlagrate jedes Mal, wenn der Herzschlagzeitpunkt berechnet wird, eine Länge einer Auslassungsperiode zu berechnen,

wobei, wenn eine Zeitdifferenz zwischen einem letzten Zeitpunkt, der aus der Abtastdatenreihe berechnet wurde, und einem unmittelbar vorangehenden Herzschlagzeitpunkt nicht größer ist als die Länge der Auslassungsperiode, die aus dem unmittelbar vorangehenden Herzschlagzeitpunkt berechnet wurde, die Herzschlagzeitpunkt-Berechnungseinheit (3) den letzten Zeitpunkt, der aus der Abtastdatenreihe berechnet wurde, nicht als einen Herzschlagzeitpunkt annimmt, **dadurch gekennzeichnet, dass** die Auslassungsperioden-Berechnungseinheit (5) konfiguriert ist, um eine Variable Y(X) derart zu berechnen, dass $Y(X) = 2(X + \Delta X)$, wenn $X + \Delta X \leq X_{max}/2$, $Y(X) - (1 + r)(X + \Delta X)$, wenn $X_{max}/2 < X + \Delta X \leq X_{max}/(1 + r)$, und $Y(X) - Y_{max}$, wenn $X + \Delta X > X_{max}/(1 + r)$, und, um die Länge der Auslassungsperiode auf der Grundlage eines Kehrwerts der Variablen Y(X) zu berechnen, wobei X die Herzschlagrate darstellt, $X_{max}$ einen vorbestimmten möglichen oberen Grenzwert der Herzschlagrate X darstellt, $\Delta X$ eine Konstante ist, die eine Variationskomponente der Herzschlagrate X darstellt, die durch eine Variation des Auftrittsintervalls einer R-Welle verursacht wird, und r eine Konstante zwischen nicht weniger als 0 und weniger als 1 ist.

7. Herzschlag-Detektionsvorrichtung, umfassend:

eine Herzschlagzeitpunkt-Berechnungseinheit (3), die konfiguriert ist, um einen Herzschlagzeitpunkt aus einer Abtastdatenreihe einer elektrokardiographischen Wellenform eines lebenden Körpers zu berechnen;
eine Herzschlagraten-Berechnungseinheit (4), die konfiguriert ist, um für jeden Herzschlagzeitpunkt eine Herzschlagrate aus dem von der Herzschlagzeitpunkt-Berechnungseinheit (3) berechneten Herzschlagzeitpunkt zu berechnen; und
eine Auslassungsperioden-Berechnungseinheit (5), die konfiguriert ist, um auf der Grundlage der von der Herzschlagraten-Berechnungseinheit (4) berechneten Herzschlagrate jedes Mal, wenn der Herzschlagzeitpunkt berechnet wird, eine Länge einer Auslassungsperiode zu berechnen,
wobei, wenn eine Zeitdifferenz zwischen einem letzten Zeitpunkt, der aus der Abtastdatenreihe berechnet wurde, und einem unmittelbar vorangehenden Herzschlagzeitpunkt nicht länger ist als die Länge der Auslassungsperiode, die aus dem unmittelbar vorangehenden Herzschlagzeitpunkt berechnet wurde, die Herzschlagzeitpunkt-Berechnungseinheit (3) den letzten Zeitpunkt, der aus der Abtastdatenreihe berechnet wurde, nicht als einen Herzschlagzeitpunkt annimmt, **dadurch gekennzeichnet, dass** die Auslassungsperioden-Berechnungseinheit (5) konfiguriert ist, um die Variable Y(X) so zu berechnen, dass $Y(X) = 2X$, wenn $X \leq X_{max}/2$, $Y(X) = (1 + r)X$, wenn $X_{max}/2 < X \leq X_{max}/(1 + r)$, und $Y(X) = X_{max}$, wenn $X > X_{max}/(1 + r)$, und die Länge der Auslassungsperiode auf der Grundlage eines Kehrwerts der Variablen Y(X) zu berechnen, wobei X die Herzschlagrate darstellt, $X_{max}$ einen vorbestimmten möglichen oberen Grenzwert der Herzschlagrate X darstellt und r eine Konstante zwischen nicht weniger als 0 und weniger als 1 ist.

8. Herzschlag-Detektionsvorrichtung, umfassend:

eine Herzschlagzeitpunkt-Berechnungseinheit (3), die konfiguriert ist, um einen Herzschlagzeitpunkt aus einer Abtastdatenreihe einer elektrokardiographischen Wellenform eines lebenden Körpers zu berechnen;
eine Herzschlagraten-Berechnungseinheit (4), die konfiguriert ist, um für jeden Herzschlagzeitpunkt eine Herzschlagrate aus dem von der Herzschlagzeitpunkt-Berechnungseinheit (3) berechneten Herzschlagzeitpunkt zu berechnen; und
eine Auslassungsperioden-Berechnungseinheit (5), die konfiguriert ist, um der Grundlage der von der Herzschlagraten-Berechnungseinheit (4) berechneten Herzschlagrate jedes Mal, wenn der Herzschlagzeitpunkt berechnet wird, eine Länge einer Auslassungsperiode zu berechnen,
wobei, wenn eine Zeitdifferenz zwischen einem letzten Zeitpunkt, der aus der Abtastdatenreihe berechnet wurde, und einem unmittelbar vorangehenden Herzschlagzeitpunkt nicht länger ist als die Länge der Auslassungsperiode, die aus dem unmittelbar vorangehenden Herzschlagzeitpunkt berechnet wurde, die Herzschlagzeitpunkt-Berechnungseinheit (3) den letzten Zeitpunkt, der aus der Abtastdatenreihe berechnet wurde, nicht als einen Herzschlagzeitpunkt annimmt, **dadurch gekennzeichnet, dass** die Auslassungsperioden-Berechnungseinheit (5) konfiguriert ist, um die Variable Y(X) so zu berechnen, dass $Y(X) - (n + 1)X$ ist, wenn $X \leq X_{max}/(n + 1)$, $Y(X) - (n + r)X$ wenn $X_{max}/(n + 1) < X \leq X_{max}/(n + r)$, $Y(X) - (m + r)X$ wenn $X_{max}/(m + 1 + r) < X \leq X_{max}/(m + r)$, und $Y(X) - X_{max}$ wenn $X > X_{max}/(1 + r)$, und die Länge der Auslassungsperiode auf der Grundlage eines Kehrwerts der Variablen Y(X) zu berechnen, wobei X die Herzschlagrate darstellt, $X_{max}$ einen vorbestimmten möglichen oberen Grenzwert der Herzschlagrate X darstellt, n eine ganze Zahl nicht kleiner als 2 ist, m jede ganze Zahl von 1 bis n-1 darstellt und r eine Konstante zwischen nicht kleiner als 0 und kleiner als 1 ist.

9. Herzschlag-Detektionsvorrichtung, umfassend:

eine Herzschlagzeitpunkt-Berechnungseinheit (3), die konfiguriert ist, um einen Herzschlagzeitpunkt aus einer Abtastdatenreihe einer elektrokardiographischen Wellenform eines lebenden Körpers zu berechnen;

eine Herzschlagraten-Berechnungseinheit (4), die konfiguriert ist, um für jeden Herzschlagzeitpunkt eine Herzschlagrate aus dem von der Herzschlagzeitpunkt-Berechnungseinheit (3) berechneten Herzschlagzeitpunkt zu berechnen; und

eine Auslassungsperioden-Berechnungseinheit (5), die konfiguriert ist, um auf der Grundlage der von der Herzschlagraten-Berechnungseinheit (4) berechneten Herzschlagrate jedes Mal, wenn der Herzschlagzeitpunkt berechnet wird, eine Länge einer Auslassungsperiode zu berechnen,

wobei, wenn eine Zeitdifferenz zwischen einem letzten Zeitpunkt, der aus der Abtastdatenreihe berechnet wurde, und einem unmittelbar vorangehenden Herzschlagzeitpunkt nicht länger ist als die Länge der Auslassungsperiode, die aus dem unmittelbar vorangehenden Herzschlagzeitpunkt berechnet wurde, die Herzschlagzeitpunkt-Berechnungseinheit (3) den letzten Zeitpunkt, der aus der Abtastdatenreihe berechnet wurde, nicht als einen Herzschlagzeitpunkt annimmt, **dadurch gekennzeichnet, dass**

die Auslassungsperioden-Berechnungseinheit (5) konfiguriert ist, um die Variable Y(X) so zu berechnen, dass $Y(X) - (n + 1)(X + \Delta X)$, wenn $X + \Delta X \leq X_{max}/(n + 1)$, $Y(X) - (n + r)(X + \Delta X)$, wenn $X_{max}/(n + 1) < X + \Delta X \leq X_{max}/(n + r)$, $Y(X) - (m + r)(X + \Delta X)$, wenn $X_{max}/(m + 1 + r) < X + \Delta X \leq X_{max}/(m + r)$, und $Y(X) - X_{max}$, wenn $X + \Delta X > X_{max}/(1 + r)$, und, die Länge der Auslassungsperiode auf der Grundlage eines Kehrwerts der Variablen $Y(X)$ zu berechnen, wobei X die Herzschlagrate darstellt, $X_{max}$ einen vorbestimmten möglichen oberen Grenzwert der Herzschlagrate X darstellt, $\Delta X$ eine Konstante ist, die eine Variationskomponente der Herzschlagrate X darstellt, die durch eine Variation im Auftrittsintervall einer R-Welle verursacht wird, n eine ganze Zahl nicht kleiner als 2 ist, m jede ganze Zahl von 1 bis n-1 darstellt, und r eine Konstante zwischen nicht kleiner als 0 und kleiner als 1 ist.

**10.** Herzschlag-Detektionsvorrichtung, umfassend:

eine Herzschlagzeitpunkt-Berechnungseinheit (3), die konfiguriert ist, um einen Herzschlagzeitpunkt aus einer Abtastdatenreihe einer elektrokardiographischen Wellenform eines lebenden Körpers zu berechnen;

eine Herzschlagraten-Berechnungseinheit (4), die konfiguriert ist, um für jeden Herzschlagzeitpunkt eine Herzschlagrate aus dem von der Herzschlagzeitpunkt-Berechnungseinheit (3) berechneten Herzschlagzeitpunkt zu berechnen; und

eine Auslassungsperioden-Berechnungseinheit (5), die konfiguriert ist, um auf der Grundlage der von der Herzschlagraten-Berechnungseinheit (4) berechneten Herzschlagrate jedes Mal, wenn der Herzschlagzeitpunkt berechnet wird, eine Länge einer Auslassungsperiode zu berechnen,

wobei, wenn eine Zeitdifferenz zwischen einem letzten Zeitpunkt, der aus der Abtastdatenreihe berechnet wurde, und einem unmittelbar vorangehenden Herzschlagzeitpunkt nicht länger ist als die Länge der Auslassungsperiode, die aus dem unmittelbar vorangehenden Herzschlagzeitpunkt berechnet wurde, die Herzschlagzeitpunkt-Berechnungseinheit (3) den letzten Zeitpunkt, der aus der Abtastdatenreihe berechnet wurde, nicht als einen Herzschlagzeitpunkt annimmt, **dadurch gekennzeichnet, dass**

die Auslassungsperioden-Berechnungseinheit (5) konfiguriert ist, um die Variable Y(X) so zu berechnen, dass $Y(X) - X + \Delta X$, wenn $X + \Delta X \leq X_{max}$ ist, und $Y(X) - X_{max}$, wenn $X + \Delta X > X_{max}$ ist, und um die Länge der Auslassungsperiode basierend auf einem Kehrwert der Variable $Y(X)$ zu berechnen, wobei X die Herzschlagrate darstellt, $X_{max}$ einen vorbestimmten möglichen oberen Grenzwert der Herzschlagrate X darstellt, $\Delta X$ eine Konstante ist, die eine Variationskomponente der Herzschlagrate X darstellt, die durch eine Variation im Auftrittsintervall einer R-Welle verursacht wird.

**11.** Programm zur Detektion von Herzschlägen, das Befehle enthält, die, wenn sie auf einem Computer ausgeführt werden, den Computer veranlassen, das Verfahren nach einem der Ansprüche 1-5 auszuführen.

**Revendications**

**1.** Procédé de détection de battement cardiaque mis en oeuvre par ordinateur comprenant :

une première étape (S1) de calcul d'un temps de battement cardiaque à partir d'une chaîne de données d'échantillonnage d'une forme d'onde électrocardiographique d'un corps vivant ;

une deuxième étape (S4) de calcul, pour chaque temps de battement cardiaque, d'une fréquence cardiaque à partir du temps de battement cardiaque calculé à la première étape ; et

une troisième étape (S5) de calcul, sur la base de la fréquence cardiaque calculée a la deuxième étape, de la durée d'une période de saut à chaque fois que le temps de battement cardiaque est calculé,

dans lequel la première étape comporte une étape où, si une différence temporelle entre un dernier temps calculé à partir de la chaîne de données d'échantillonnage et un temps de battement cardiaque immédiatement précédent n'est pas supérieure à la durée de la période de saut calculée à partir du temps de battement cardiaque immédiatement précédent, le dernier temps calculé à partir de la chaîne de données d'échantillonnage n'est pas adopté en tant que temps de battement cardiaque, **caractérisé en ce que**

la troisième étape comporte une étape où une variable $Y(X)$ est calculée de sorte que $Y(X) - 2(X + \Delta X)$ si $X + \Delta X \leq X_{max}/2$, $Y(X) - (1 + r)(X + \Delta X)$ si $X_{max}/2 < X + \Delta X \leq X_{max}/(1 + r)$, et $Y(X) - Y_{max}$ si $X + \Delta X > X_{max}/(1 + r)$, et la durée de la période de saut est calculée sur la base d'une réciproque de la variable $Y(X)$, dans lequel $X$ représente la fréquence cardiaque, $X_{max}$ représente une valeur limite supérieure possible prédéterminée de la fréquence cardiaque $X$, $\Delta X$ est une constante qui représente une composante de variation de la fréquence cardiaque $X$ causée par une variation de l'intervalle d'apparition d'une onde R, et $r$ est une constante supérieure ou égale à 0 et inférieure à 1.

2. Procédé de détection de battement cardiaque mis en oeuvre par ordinateur comprenant :

une première étape (S1) de calcul d'un temps de battement cardiaque à partir d'une chaîne de données d'échantillonnage d'une forme d'onde électrocardiographique d'un corps vivant ;
une deuxième étape (S4) de calcul, pour chaque temps de battement cardiaque, d'une fréquence cardiaque à partir du temps de battement cardiaque calculé à la première étape ; et
une troisième étape (S5) de calcul, sur la base de la fréquence cardiaque calculée a la deuxième étape, de la durée d'une période de saut à chaque fois que le temps de battement cardiaque est calculé,

dans lequel la première étape comporte une étape où, si une différence temporelle entre un dernier temps calculé à partir de la chaîne de données d'échantillonnage et un temps de battement cardiaque immédiatement précédent n'est pas supérieure à la durée de la période de saut calculée à partir du temps de battement cardiaque immédiatement précédent, le dernier temps calculé à partir de la chaîne de données d'échantillonnage n'est pas adopté en tant que temps de battement cardiaque, **caractérisé en ce que**

la troisième étape comporte une étape où la variable $Y(X)$ est calculée de sorte que $Y(X) - 2X$ si $X \leq X_{max}/2$, $Y(X) - (1 + r)X$ si $X_{max}/2 < X \leq X_{max}/(1 + r)$, et $Y(X) - X_{max}$ si $X > X_{max}/(1 + r)$, et la durée de la période de saut est calculée sur la base d'une réciproque de la variable $Y(X)$, dans lequel $X$ représente la fréquence cardiaque, $X_{max}$ représente une valeur limite supérieure possible prédéterminée de la fréquence cardiaque $X$, et $r$ est une constante supérieure ou égale à 0 et inférieure à 1.

3. Procédé de détection de battement cardiaque mis en oeuvre par ordinateur comprenant :

une première étape (S1) de calcul d'un temps de battement cardiaque à partir d'une chaîne de données d'échantillonnage d'une forme d'onde électrocardiographique d'un corps vivant ;
une deuxième étape (S4) de calcul, pour chaque temps de battement cardiaque, d'une fréquence cardiaque à partir du temps de battement cardiaque calculé à la première étape ; et
une troisième étape (S5) de calcul, sur la base de la fréquence cardiaque calculée a la deuxième étape, de la durée d'une période de saut à chaque fois que le temps de battement cardiaque est calculé,

dans lequel la première étape comporte une étape où, si une différence temporelle entre un dernier temps calculé à partir de la chaîne de données d'échantillonnage et un temps de battement cardiaque immédiatement précédent n'est pas supérieure à la durée de la période de saut calculée à partir du temps de battement cardiaque immédiatement précédent, le dernier temps calculé à partir de la chaîne de données d'échantillonnage n'est pas adopté en tant que temps de battement cardiaque, **caractérisé en ce que**

la troisième étape comporte une étape où la variable $Y(X)$ est calculée de sorte que $Y(X) - (n + 1)X$ si $X \leq X_{max}/(n + 1)$, $Y(X) - (n + r)X$ si $X_{max}/(n + 1) < X \leq X_{max}/(n + r)$, $Y(X) - (m + r)X$ si $X_{max}/(m + 1 + r) < X \leq X_{max}/(m + r)$, et $Y(X) = X_{max}$ si $X > X_{max}/(1 + r)$, et la durée de la période de saut est calculée sur la base d'une réciproque de la variable $Y(X)$, dans lequel $X$ représente la fréquence cardiaque, $X_{max}$ représente une valeur limite supérieure possible prédéterminée de la fréquence cardiaque $X$, $n$ est un nombre entier supérieur ou égal à 2, , $m$ représente chaque nombre entier de 1 à $n - 1$, et $r$ est une constante supérieure ou égale à 0 et inférieure à 1.

4. Procédé de détection de battement cardiaque mis en oeuvre par ordinateur comprenant :

une première étape (S1) de calcul d'un temps de battement cardiaque à partir d'une chaîne de données d'échantillonnage d'une forme d'onde électrocardiographique d'un corps vivant ;
une deuxième étape (S4) de calcul, pour chaque temps de battement cardiaque, d'une fréquence cardiaque à partir du temps de battement cardiaque calculé à la première étape ; et

une troisième étape (S5) de calcul, sur la base de la fréquence cardiaque calculée a la deuxième étape, de la durée d'une période de saut à chaque fois que le temps de battement cardiaque est calculé,

dans lequel la première étape comporte une étape où, si une différence temporelle entre un dernier temps calculé à partir de la chaîne de données d'échantillonnage et un temps de battement cardiaque immédiatement précédent n'est pas supérieure à la durée de la période de saut calculée à partir du temps de battement cardiaque immédiatement précédent, le dernier temps calculé à partir de la chaîne de données d'échantillonnage n'est pas adopté en tant que temps de battement cardiaque, **caractérisé en ce que**

la troisième étape comporte une étape où la variable $Y(X)$ est calculée de sorte que $Y(X) - (n + 1)(X + \Delta X)$ si $X + \Delta X \leq X_{max}/(n + 1)$, $Y(X) - (n + r)(X + \Delta X)$ si $X_{max}/(n + 1) < X + \Delta X \leq X_{max}/(n + r)$, $Y(X) - (m + r)(X + \Delta X)$ si $X_{max}/(m + 1 + r) < X + \Delta X \leq X_{max}/(m + r)$, et $Y(X) - X_{max}$ si $X + \Delta X > X_{max}/(1 + r)$, et la durée de la période de saut est calculée sur la base d'une réciproque de la variable $Y(X)$, dans lequel $X$ représente la fréquence cardiaque, $X_{max}$ représente une valeur limite supérieure possible prédéterminée de la fréquence cardiaque $X$, $\Delta X$ est une constante qui représente une composante de variation de la fréquence cardiaque $X$ causée par une variation de l'intervalle d'apparition d'une onde R, $n$ est un nombre entier supérieur ou égal à 2, $m$ représente chaque nombre entier de 1 à n-1, et $r$ est une constante supérieure ou égale à 0 et inférieure à 1.

5. Procédé de détection de battement cardiaque mis en oeuvre par ordinateur comprenant :

une première étape (S1) de calcul d'un temps de battement cardiaque à partir d'une chaîne de données d'échantillonnage d'une forme d'onde électrocardiographique d'un corps vivant ;

une deuxième étape (S4) de calcul, pour chaque temps de battement cardiaque, d'une fréquence cardiaque à partir du temps de battement cardiaque calculé à la première étape ; et

une troisième étape (S5) de calcul, sur la base de la fréquence cardiaque calculée a la deuxième étape, de la durée d'une période de saut à chaque fois que le temps de battement cardiaque est calculé,

dans lequel la première étape comporte une étape où, si une différence temporelle entre un dernier temps calculé à partir de la chaîne de données d'échantillonnage et un temps de battement cardiaque immédiatement précédent n'est pas supérieure à la durée de la période de saut calculée à partir du temps de battement cardiaque immédiatement précédent, le dernier temps calculé à partir de la chaîne de données d'échantillonnage n'est pas adopté en tant que temps de battement cardiaque, **caractérisé en ce que**

la troisième étape comporte une étape où la variable $Y(X)$ est calculée de sorte que $Y(X) - X + \Delta X$ si $X + \Delta X \leq X_{max}$, et $Y(X) = X_{max}$ si $X + \Delta X > X_{max}$, et la durée de la période de saut est calculée sur la base d'une réciproque de la variable $Y(X)$, dans lequel $X$ représente la fréquence cardiaque, $X_{max}$ représente une valeur limite supérieure possible prédéterminée de la fréquence cardiaque $X$, $\Delta X$ est une constante qui représente une composante de variation de la fréquence cardiaque $X$ causée par une variation de l'intervalle d'apparition d'une onde R.

6. Dispositif de détection de battement cardiaque comprenant :

une unité de calcul de temps de battement cardiaque (3) configurée pour calculer un temps de battement cardiaque à partir d'une chaîne de données d'échantillonnage d'une forme d'onde électrocardiographique d'un corps vivant ;

une unité de calcul de fréquence cardiaque (4) configurée pour calculer, pour chaque temps de battement cardiaque, une fréquence cardiaque à partir du temps de battement cardiaque calculé par l'unité de calcul de temps de battement cardiaque (3) ; et

une unité de calcul de période de saut (5) configurée pour calculer, sur la base de la fréquence cardiaque calculée par l'unité de calcul de fréquence cardiaque (4), la durée d'une période de saut à chaque fois que le temps de battement cardiaque est calculé,

dans lequel, si une différence temporelle entre un dernier temps calculé à partir de la chaîne de données d'échantillonnage et un temps de battement cardiaque immédiatement précédent n'est pas supérieure à la durée de la période de saut calculée à partir du temps de battement cardiaque immédiatement précédent, l'unité de calcul de temps de battement cardiaque (3) n'adopte pas, en tant que temps de battement cardiaque, le dernier temps calculé à partir de la chaîne de données d'échantillonnage, **caractérisé en ce que**

l'unité de calcul de période de saut (5) est configurée pour calculer une variable $Y(X)$ de sorte que $Y(X) - 2(X + \Delta X)$ si $X + \Delta X \leq X_{max}/2$, $Y(X) - (1 + r)(X + \Delta X)$ si $X_{max}/2 < X + \Delta X \leq X_{max}/(1 + r)$, et $Y(X) - Y_{max}$ si $X + \Delta X > X_{max}/(1 + r)$, et pour calculer la durée de la période de saut sur la base d'une réciproque de la variable $Y(X)$, dans lequel $X$ représente la fréquence cardiaque, $X_{max}$ représente une valeur limite supérieure possible prédéterminée de la fréquence cardiaque $X$, $\Delta X$ est une constante qui représente une composante de variation de la fréquence cardiaque $X$ causée par une variation de l'intervalle d'apparition d'une onde R, et $r$ est une constante supérieure ou égale à 0 et inférieure à 1.

7. Dispositif de détection de battement cardiaque comprenant :

une unité de calcul de temps de battement cardiaque (3) configurée pour calculer un temps de battement cardiaque à partir d'une chaîne de données d'échantillonnage d'une forme d'onde électrocardiographique d'un corps vivant ;
une unité de calcul de fréquence cardiaque (4) configurée pour calculer, pour chaque temps de battement cardiaque, une fréquence cardiaque à partir du temps de battement cardiaque calculé par l'unité de calcul de temps de battement cardiaque (3) ; et
une unité de calcul de période de saut (5) configurée pour calculer, sur la base de la fréquence cardiaque calculée par l'unité de calcul de fréquence cardiaque (4), la durée d'une période de saut à chaque fois que le temps de battement cardiaque est calculé,
dans lequel, si une différence temporelle entre un dernier temps calculé à partir de la chaîne de données d'échantillonnage et un temps de battement cardiaque immédiatement précédent n'est pas supérieure à la durée de la période de saut calculée à partir du temps de battement cardiaque immédiatement précédent, l'unité de calcul de temps de battement cardiaque (3) n'adopte pas, en tant que temps de battement cardiaque, le dernier temps calculé à partir de la chaîne de données d'échantillonnage, **caractérisé en ce que**
l'unité de calcul de période de saut (5) est configurée pour calculer la variable $Y(X)$ de sorte que $Y(X) - 2X$ si $X \leq X_{max}/2$, $Y(X) = (1 + r)X$ si $X_{max}/2 < X \leq X_{max}/(1 + r)$, et $Y(X) - X_{max}$ si $X > X_{max}/(1 + r)$, et pour calculer la durée de la période de saut sur la base d'une réciproque de la variable $Y(X)$, dans lequel $X$ représente la fréquence cardiaque, $X_{max}$ représente une valeur limite supérieure possible prédéterminée de la fréquence cardiaque $X$, et $r$ est une constante supérieure ou égale à 0 et inférieure à 1.

8. Dispositif de détection de battement cardiaque comprenant :

une unité de calcul de temps de battement cardiaque (3) configurée pour calculer un temps de battement cardiaque à partir d'une chaîne de données d'échantillonnage d'une forme d'onde électrocardiographique d'un corps vivant ;
une unité de calcul de fréquence cardiaque (4) configurée pour calculer, pour chaque temps de battement cardiaque, une fréquence cardiaque à partir du temps de battement cardiaque calculé par l'unité de calcul de temps de battement cardiaque (3) ; et
une unité de calcul de période de saut (5) configurée pour calculer, sur la base de la fréquence cardiaque calculée par l'unité de calcul de fréquence cardiaque (4), la durée d'une période de saut à chaque fois que le temps de battement cardiaque est calculé,
dans lequel, si une différence temporelle entre un dernier temps calculé à partir de la chaîne de données d'échantillonnage et un temps de battement cardiaque immédiatement précédent n'est pas supérieure à la durée de la période de saut calculée à partir du temps de battement cardiaque immédiatement précédent, l'unité de calcul de temps de battement cardiaque (3) n'adopte pas, en tant que temps de battement cardiaque, le dernier temps calculé à partir de la chaîne de données d'échantillonnage, **caractérisé en ce que**
l'unité de calcul de période de saut (5) est configurée pour calculer la variable $Y(X)$ de sorte que $Y(X) - (n + 1)X$ si $X \leq X_{max}/(n + 1)$, $Y(X) - (n + r)X$ si $X_{max}/(n + 1) < X \leq X_{max}/(n + r)$, $Y(X) = (m + r)X$ si $X_{max}/(m + 1 + r) < X \leq X_{max}/(m + r)$, et $Y(X) - X_{max}$ si $X > X_{max}/(1 + r)$, et pour calculer la durée de la période de sur la base d'une réciproque de la variable $Y(X)$, dans lequel $X$ représente la fréquence cardiaque, $X_{max}$ représente une valeur limite supérieure possible prédéterminée de la fréquence cardiaque $X$, $n$ est un nombre entier supérieur ou égal à 2, , $m$ représente chaque nombre entier de 1 à n-1, et $r$ est une constante supérieure ou égale à 0 et inférieure à 1.

9. Dispositif de détection de battement cardiaque comprenant :

une unité de calcul de temps de battement cardiaque (3) configurée pour calculer un temps de battement cardiaque à partir d'une chaîne de données d'échantillonnage d'une forme d'onde électrocardiographique d'un corps vivant ;
une unité de calcul de fréquence cardiaque (4) configurée pour calculer, pour chaque temps de battement cardiaque, une fréquence cardiaque à partir du temps de battement cardiaque calculé par l'unité de calcul de temps de battement cardiaque (3) ; et
une unité de calcul de période de saut (5) configurée pour calculer, sur la base de la fréquence cardiaque calculée par l'unité de calcul de fréquence cardiaque (4), la durée d'une période de saut à chaque fois que le temps de battement cardiaque est calculé,

dans lequel, si une différence temporelle entre un dernier temps calculé à partir de la chaîne de données d'échantillonnage et un temps de battement cardiaque immédiatement précédent n'est pas supérieure à la durée de la période de saut calculée à partir du temps de battement cardiaque immédiatement précédent, l'unité de calcul de temps de battement cardiaque (3) n'adopte pas, en tant que temps de battement cardiaque, le dernier temps calculé à partir de la chaîne de données d'échantillonnage, **caractérisé en ce que** l'unité de calcul de période de saut (5) est configurée pour calculer la variable Y(X) de sorte que Y(X) - (n + 1) (X + $\Delta$X) si X + $\Delta$X $\leq$ X$_{max}$/(n + 1), Y(X) - (n + r) (X + $\Delta$X) si X$_{max}$/(n + 1) < X + $\Delta$X $\leq$ X$_{max}$/(n + r), Y(X) = (m + r) (X + $\Delta$X) si X$_{max}$/(m + 1 + r) < X + $\Delta$X $\leq$ X$_{max}$/(m + r), et Y(X) - X$_{max}$ si X + $\Delta$X > X$_{max}$/(1 + r), et pour calculer la durée de la période de saut sur la base d'une réciproque de la variable Y(X), dans lequel X représente la fréquence cardiaque, X$_{max}$ représente une valeur limite supérieure possible prédéterminée de la fréquence cardiaque X, $\Delta$X est une constante qui représente une composante de variation de la fréquence cardiaque X causée par une variation de l'intervalle d'apparition d'une onde R, n est un nombre entier supérieur ou égal à 2, , m représente chaque nombre entier de 1 à n-1, et r est une constante supérieure ou égale à 0 et inférieure à 1.

10. Dispositif de détection de battement cardiaque comprenant :

une unité de calcul de temps de battement cardiaque (3) configurée pour calculer un temps de battement cardiaque à partir d'une chaîne de données d'échantillonnage d'une forme d'onde électrocardiographique d'un corps vivant ;
une unité de calcul de fréquence cardiaque (4) configurée pour calculer, pour chaque temps de battement cardiaque, une fréquence cardiaque à partir du temps de battement cardiaque calculé par l'unité de calcul de temps de battement cardiaque (3) ; et
une unité de calcul de période de saut (5) configurée pour calculer, sur la base de la fréquence cardiaque calculée par l'unité de calcul de fréquence cardiaque (4), la durée d'une période de saut à chaque fois que le temps de battement cardiaque est calculé,
dans lequel, si une différence temporelle entre un dernier temps calculé à partir de la chaîne de données d'échantillonnage et un temps de battement cardiaque immédiatement précédent n'est pas supérieure à la durée de la période de saut calculée à partir du temps de battement cardiaque immédiatement précédent, l'unité de calcul de temps de battement cardiaque (3) n'adopte pas, en tant que temps de battement cardiaque, le dernier temps calculé à partir de la chaîne de données d'échantillonnage, **caractérisé en ce que** l'unité de calcul de période de saut (5) est configurée pour calculer la variable Y(X) de sorte que Y (X) - X + $\Delta$X si X + $\Delta$X $\leq$ X$_{max}$, et Y (X) - X$_{max}$ si X + $\Delta$X > X$_{max}$, et pour calculer la durée de la période de saut sur la base d'une réciproque de la variable Y(X), dans lequel X représente la fréquence cardiaque, X$_{max}$ représente une valeur limite supérieure possible prédéterminée de la fréquence cardiaque X, $\Delta$X est une constante qui représente une composante de variation de la fréquence cardiaque X causée par une variation de l'intervalle d'apparition d'une onde R.

11. Programme de détection de battement cardiaque comprenant des instructions qui, lorsqu'elles ont exécutées sur un ordinateur, amènent ledit ordinateur à exécuter le procédé de l'une des revendications 1 à 5.

# FIG. 1

ELECTRO-CARDIOGRAPH 1 → STORAGE UNIT 2 → HEARTBEAT TIME CALCULATION UNIT 3 → HEART RATE CALCULATION UNIT 4

SKIP PERIOD CALCULATION UNIT 5

# FIG. 2

START

CALCULATE HEARTBEAT TIME — S1

IS TIME DIFFERENCE BETWEEN LATEST TIME AND IMMEDIATELY PRECEDINGLY CALCULATED HEARTBEAT TIME LONGER THAN SKIP PERIOD? — S2

NO

YES

CONFIRM HEARTBEAT TIME — S3

CALCULATE HEART RATE — S4

CALCULATE LENGTH OF SKIP PERIOD — S5

END

# FIG. 3

# FIG. 4

# FIG. 5

_3_

```
                                          ┌──────────────────────┐
                                       40 │  TIME DIFFERENCE     │
                                          │       VALUE          │
                                          │  CALCULATION UNIT    │
                                          └──────────────────────┘
                                                   ▲   │
                                                   │   ▼
  ┌──────────────────────┐             ┌──────────────────────┐
42│   INDEX VALUE         │◄──────────► │    MINIMUM           │ 41
  │   CALCULATION         │             │     VALUE            │
  │      UNIT             │             │  ACQUISITION         │
  └──────────────────────┘             │     UNIT             │
          ▲   │                        └──────────────────────┘
          │   ▼  43
  ┌──────────────────────┐
  │      TIME            │
  │    DECISION          │
  │      UNIT            │
  └──────────────────────┘
```

## FIG. 6

# FIG.7

# FIG. 8

## FIG. 9

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2015156936 A
- JP 2017029628 A
- JP 2017042388 A
- JP 2017150156 A
- US 20090082682 A1
- US 20090171228 A1
- JP 2018011819 A